# EUROPEAN PATENT APPLICATION

(11) **EP 1 956 098 A1**
(43) Date of publication of application: **13.08.2008**
(21) Application number: 08001288.3
(22) Date of filing: 24.01.2008
(51) Int. Cl.: C12Q 1/68

(54) **Method of detecting gene mutation**

(30) Priority: 09.02.2007 JP 2007031318
(71) Applicant: Kabushiki Kaisha Toshiba, Minato-ku, Tokyo 105-8001 (JP)
(72) Inventor: Nakamura, Naoko, Minato-ku, Tokyo 105-8001 (JP)
(74) Representative: HOFFMANN EITLE

(57) **Abstract**

There is provided a method of detecting a gene insertion or deletion mutation, includes the steps of reacting a target nucleic acid with a wild-type probe and a mutant-type probe, detecting the amount of the target nucleic acid bound to the each probes, and comparing the amount of the target nucleic acid bound to the wild-type probe with the amount of the target nucleic acid bound to the mutant-type probe.

## Description

The present invention relates to a method of detecting gene insertion and deletion mutations.

In recent years, techniques of detecting genetic polymorphism have been rapidly developed. A method of using a probe immobilized on a substrate is one of such techniques. In this method, a target nucleic acid is detected by hybridization with the probe.

The genetic polymorphism includes an insertion mutation, a deletion mutation, etc., in addition to single base substitution. There are many reports on detection of single base substitution by using the above-mentioned method. However, there are few reports on detection of an insertion mutation and a deletion mutation by using the above-mentioned method (see, for example, Nat Genet. 14, 441-7, 1996 and Nucleic Acid Res. 33 e33, 2005).

When an insertion mutation or a deletion mutation is detected with a probe, hybridization of the probe with the target nucleic acid might occur with a mutation site being looped out, and thus unspecific hybridization occurs. It causes of a problem that a mutation cannot be accurately detected. Therefore, there is a need for development of a detection method of using an immobilized probe capable of accurately detecting insertion and deletion mutations.

One object of the present invention is to provide a method of detecting a gene mutation by using a probe, which can accurately detect an insertion mutation and a deletion mutation.

According to one aspect of the invention, there is provided a method of detecting a gene insertion mutation, comprising the steps of reacting a target nucleic acid with a wild-type probe and a mutant-type probe, detecting the amount of the target nucleic acid which has been bound to each of the probes, and comparing the amount of the target nucleic acid bound to the wild-type probe with the amount of the target nucleic acid bound to the mutant-type probe.

The invention can be more fully understood from the following detailed description when taken in conjunction with the accompanying drawings, in which:
FIG. 1A is a schematic diagram of the reaction of an insertion mutant-type target nucleic acid with a probe;
FIG. 1B is a schematic diagram of the reaction of an insertion mutant-type target nucleic acid with a probe when the mutation is positioned outside of the center;
FIG. 2A is a schematic diagram of the reaction of the wild-type target nucleic acid and the wild-type probe;
FIG. 2B is a schematic diagram of the reaction of an insertion mutant-type target nucleic acid and the wild-type probe;
FIG. 2C is a schematic diagram of the reaction of the wild-type target nucleic acid and the mutant-type probe;
FIG. 2D is a schematic diagram of the reaction of an insertion mutant-type target nucleic acid and the mutant-type probe;
FIG. 3A is a schematic diagram of the reaction of an wild-type target nucleic acid and the wild-type probe;
FIG. 3B is a schematic diagram of the reaction of an deletion mutant-type target nucleic acid and the wild-type probe;
FIG. 3C is a schematic diagram of the reaction of an wild-type target nucleic acid and the mutant-type probe;
FIG. 3D is a schematic diagram of the reaction of an deletion mutant-type target nucleic acid and the mutant-type probe;
FIG. 4 is a schematic diagram for showing the positions of primers used in LAMP method;
FIG. 5 shows a sequence of CYP2D6 gene;
FIG. 6 is a schematic diagram of the reaction between CYP2D6*18 and a wild-type probe;
FIG. 7 is a schematic diagram of the reaction between CYP2D6 and a mutant-type probe;
FIG. 8A shows the result of detection of CYP2D6 with each probe; and
FIG. 8B shows the result of detection of CYP2D6*18 with each probe.

The present invention provides a method of detecting gene insertion and deletion mutations. According to the present invention, whether an objective gene is wild-type or mutant-type can be determined. The term "wild-type" as used herein means a genotype appearing at high frequency, while the term "mutant-type" as used herein means a genotype appearing at low frequency. In the present context, a nucleic acid as an object of detection is referred to as target nucleic acid. A probe having a sequence complementary to a wild-type target nucleic acid is referred to as wild-type probe, while a probe having a sequence complementary to a mutant-type target nucleic acid is referred to as mutant-type probe.

A probe used in the conventional methods has a mutation located in the vicinity of the center of the probe. For example, in Nat Genet. 14, 441-7, 1996., a probe of 20 bases is used, and a mutation site is located at the ninth base from the 3'-terminal of the probe. In Nucleic Acid Res. 33 e33, 2005., a probe of 25 bases is used, and a mutation site is located at the thirteenth base from the 3'-terminal of the probe.

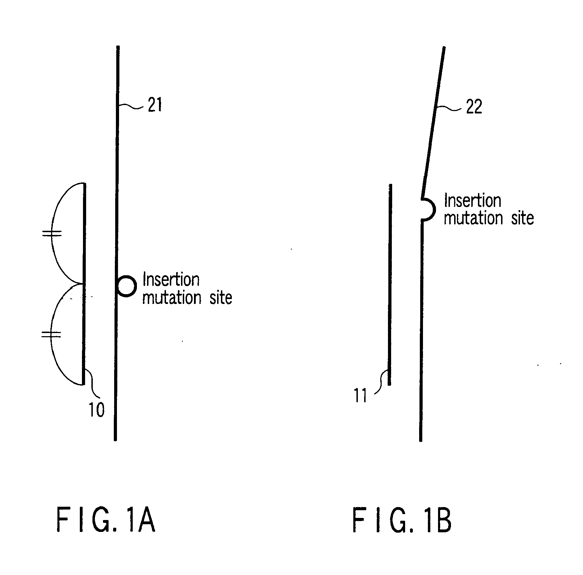

However, when a mutation site is located in the vicinity of the center of a probe, the probe will bind to a target nucleic acid regardless of the presence or non-presence of the mutation in the target nucleic acid. An schematic example is shown in FIG. 1A. FIG. 1A shows the reaction between an insertion mutant-type target nucleic acid 21 and a wild-type probe 10. The mutant-type target nucleic acid 21 will originally not bind to the wild-type probe 10 because their sequences are not complementary to each other. As shown in FIG. 1A, however, the target nucleic acid 21 will bind to the probe 10, with its insertion mutation site being looped out. Wherein, the chain length of from one end of the probe 10 to the mutation site and from the other end of the probe 10 to the mutation site are approximately equal, thus making binding stable in both sides. As a result, the target nucleic acid 21, though being mutant-type, binds to the wild-type probe 10 to cause an error of determination. In the case of a target nucleic acid with deletion mutation, on the other hand, the wild-type probe will bind stably to its target nucleic acid, with a site correspond to the mutation site of the target being looped out.

It follows that as shown in the present invention, the mutation site can be positioned outside of the center of the probe to reduce unspecific binding. When the mutation is positioned outside of the center of the probe 11 as shown in FIG. 1B, the region from one end of the probe to the mutation site will be shorter than the other region. Accordingly, the binding in the shorter region will be destabilized, thus allowing this region to be easily separated from the target nucleic acid 22. Unspecific binding can thereby be reduced. By using the probe having a mutation site at a suitable position according to the present invention, detection accuracy can be improved.

A suitable probe can be determined for example by using Tm as follows:
First, a probe for detecting an insertion mutation is described.

A wild-type probe for detecting an insertion mutation is suitably a probe wherein the ratio of Tm value of the sequence from one end of the probe to the mutation site to Tm value of the sequence from the other end of the probe to the mutation site is 1:2 or more. The mutation site about the wild-type probe as used herein means a site corresponds to a site which the mutation is present in the target nucleic acid.

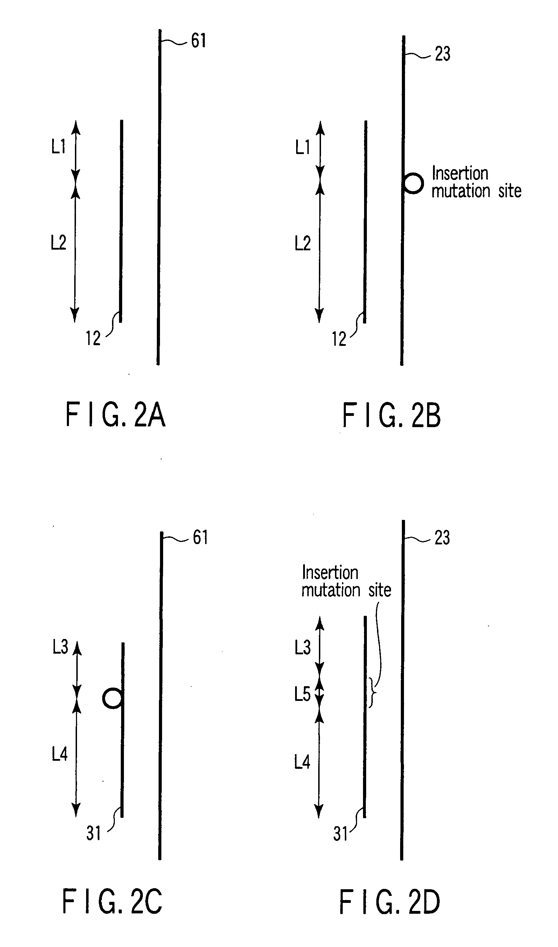

An example of the wild-type probe 12 is shown in FIG. 2A for detection of an wild-type target nucleic acid 61 and in FIG. 2B for detection of an insertion mutant-type target nucleic acid 23. A region from one end of the wild-type probe 12 to the mutation site having lower Tm value is referred to as L1, and the other region of the wild-type probe 12 is referred to as L2. Preferably the probe used in the method has the ratio of Tm value of L1 to Tm value of L2 ranges from 1:2 or more. The ratio of Tm value of L1 to L2 is more preferably 1:3 or more. From the viewpoint of improving detection accuracy, it is preferable the ratio of Tm value of L1 to Tm value of L2 ranges from 1:15 or less.

A mutant-type probe used for detecting an insertion mutation is desirably a probe wherein the ratio of Tm value of the sequence from one end of the probe to the mutation site to Tm value of the sequence from the other end of the probe to the mutation site is 1:2 or more, when the number of inserted bases is 3 or less. Examples of the mutant-type probe are shown in FIGS. 2C and 2D. In FIG. 2C, a region from one end of the mutant-type probe 31 to the mutation site having lower Tm value is referred to as L3, and the other region of the mutant-type probe 31 is referred to as L4. Further, the inserted region of the probe is referred to as L5. When the mutant-type probe 31 binds to a wild-type target nucleic acid 61 as shown in FIG. 2C, L3 + L4 region of the mutant-type probe 31 binds to the nucleic acid 61. Then the probe used in this method is preferably a probe when the number of inserted bases is 3 or less, having the ratio of Tm value of L3 to Tm value of L4 ranges from 1:2 or more. The ratio of Tm value of L3 to L4 is more preferably 1:3 or more. From the viewpoint of improving detection accuracy, it is preferable the ratio of Tm value of L3 to Tm value of L4 ranges from 1:15 or less.

When the mutation site reaches one end of the mutant-type probe, L3 does not exist. When the number of inserted bases is large, for example when the number of inserted bases is 4 or more, the length of L3 + L4 region will be shorter. In this case, the Tm of L3 + L4 region will be small to make binding unstable, thus decreasing the unspecific binding of the probe to the target nucleic acid. However, when the number of inserted bases is 3 or less, unspecific binding will easily occur; hence, when the number of inserted bases is 3 or less, the probe according the invention is advantageous.

Then, a probe for detecting a deletion mutation is described.

A mutant-type probe for detecting a deletion mutation is suitably a probe wherein the ratio of Tm value of the sequence from one end of the probe to the mutation site to Tm value of the sequence from the other end of the probe to the mutation site is 1:2 or more. The mutation site about the wild-type probe as used herein means a site corresponds to a site which the mutation is present in the target nucleic acid.

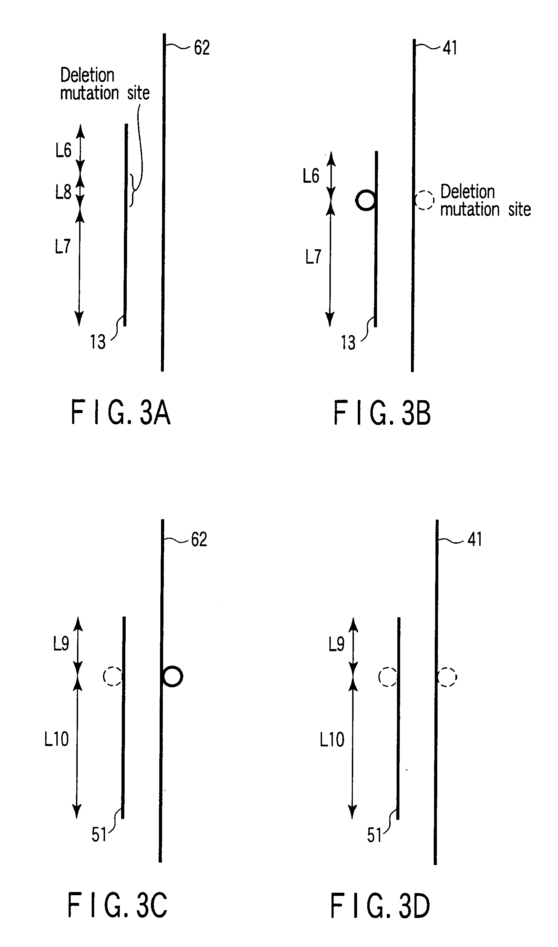

An example of the mutant-type probe for detection of a deletion mutation is shown in FIGS. 3C and 3D. In FIGS. 3C and 3D, a region from one end of the mutant-type probe 51 to the mutation site having lower Tm value is referred to as L9, and the other region of the mutant-type probe 51 is referred to as L10. Preferably the probe used in the method has the ratio of Tm value of L9 to Tm value of L10 ranges from 1:2 or more. The ratio of Tm value of L9 to L10 is more preferably 1:3 or more. From the viewpoint of improving detection accuracy, it is preferable the ratio of Tm value of L9 to Tm value of L10 ranges from 1:15 or less.

A wild-type probe used for detecting a deletion mutation is desirably a probe wherein the ratio of Tm value of the sequence from one end of the probe to the mutation site to Tm value of the sequence from the other end of the probe to the mutation site is 1:2 or more, when the number of deleted bases is 3 or less. An example of the wild-type probe 13 is shown in FIG. 3A for detection of a wild-type target nucleic acid 62 and in FIG. 3B for detection of an deletion mutant-type target nucleic acid 41. In FIG. 3B, a region from one end of the wild-type probe 13 to the mutation site having lower Tm value is referred to as L6, and the other region of the wild-type probe 13 is referred to as L7. Further, the deleted region of the probe is referred to as L8. When the wild-type probe 13 binds to a mutant-type target nucleic acid 41 as shown in FIG. 3B, L6 + L7 region of the wild-type probe 13 binds to the nucleic acid 41. Then the probe used in this method is preferably a probe when the number of deleted bases is 3 or less, having the ratio of Tm value of L6 to Tm value of L7 ranges from 1:2 or more. The ratio of Tm value of L6 to L7 is more preferably 1:3 or more. From the viewpoint of improving detection accuracy, it is preferable the ratio of Tm value of L6 to Tm value of L7 ranges from 1:15 or less.

When the mutation site reaches one end of the wild-type probe, L6 does not exist as is the case with the above description of the insertion mutation. When the number of deleted bases is large, for example when the number of deleted bases is 4 or more, the length of L6 + L7 region will be very shorter. In this case, the Tm of L6 + L7 region will be small to make binding unstable, thus decreasing the unspecific binding of the probe to the target nucleic acid. However, when the number of deleted bases is 3 or less, unspecific binding will easily occur; hence, when the number of deleted bases is 3 or less, the probe according the invention is advantageous.

For use in detection, the Tm value of the wild-type probe as a whole is preferably almost equal to the Tm value of the mutant-type probe. The difference in Tm values thereof is preferably within 10°C.

The Tm value may be calculated by any known method. The Wallace method suitable for handling short base sequences (see Nucleic Acid Res. 6, 3543-3557, 1979) is preferably used. In the Wallace method, Tm value is calculated on the assumption that the bonding force of guanine or cytosine is 4°C and the bonding force of adenine or thymine is 2°C. As other methods, a nearest base pair method or GC% method can be used.

In the method of detecting a mutation according to the present invention, the probe described above is immobilized on a support. The wild-type probe and the mutant-type probe are immobilized preferably on the same support. First, a sample solution containing a target nucleic acid is reacted with the immobilized wild-type and mutant-type probes respectively. Then, the amount of the target nucleic acid bound to each probe is detected. Then, the amount of the target nucleic acid bound to the wild-type probe is compared with the amount of the target nucleic acid bound to the mutant-type probe. When a larger amount of the target nucleic acid was bound to the mutant-type probe, it is determined that the genotype of the target nucleic acid is mutant-type. On the other hand, when a larger amount of the target nucleic acid was bound to the wild-type probe, it is determined that the genotype of the target nucleic acid is wild-type. By comparing the detection results in this manner, the mutation of the target nucleic acid can be detected.

### <Nucleic acid>

The term "nucleic acid" used in this specification is intended to encompass substances such as DNA, RNA, PNA, S-oligo and methyl phosphonate oligo, a partial structure of which can be expressed in base sequence.

### <Target nucleic acid>

The target nucleic acid in the present invention may be a naturally occurring nucleic acid or an artificially produced nucleic acid analog. The naturally occurring nucleic acid includes individual genomic DNA, genomic RNA and mRNA. The individual includes, but is not limited to, humans, non-human animals, plants, viruses, and microorganisms such as bacteria, yeasts and mycoplasma.

These nucleic acids are extracted from samples collected from individuals, for example, blood, serum, leucocytes, urine, feces, semen, saliva, tissues, biopsy, oral mucosa, cultured cells, expectorated sputum, etc. Alternatively, the nucleic acid is extracted directly from microorganisms. Extraction of the nucleic acid may be carried out by using, for example, commercial nucleic acid extraction kits QIAamp (QIAGEN) or SMITEST (Sumitomo Metal Industries, Ltd.).

A solution containing a nucleic acid extracted from an individual sample or a microorganism is referred to as a sample solution. The sample solution can be subjected to any treatments such as amplification and purification. A treated sample solution is subjected to the detection method of the present invention.

The extracted nucleic acid is preferably amplified by known amplification techniques. Examples of the amplification method include, but are not limited to, polymerase chain reaction (PCR), loop mediated isothermal amplification method (LAMP method), isothermal and chimeric primer-initiated amplification of nucleic acids (ICAN method), nucleic acid sequence-based amplification method (NASBA method), strand displacement amplification (SDA method) and ligase chain reaction (LCR method) and rolling circle amplification method (RCA method). By specifically amplifying a region desired to be detected, the region of the nucleic acid subjected to detection is thereby limited. By doing so, detection is made feasible even if specificity cannot be attained with only the probe.

The target nucleic acid or the amplification product is made single-stranded in order to bind efficiently to the probe. Examples of the method of making the nucleic acid single-stranded include, but are not limited to, a method of using beads or an enzyme and a method of transcriptional reaction with T7 RNA polymerase. A sample that is an amplification product amplified by LAMP method or ICAN method and not required to be single-stranded is subjected directly to the next step.

The amplified target nucleic acid preferably has a stem loop structure because the step of making it single-stranded is not necessary. The amplification product having a stem loop structure is advantageous in hybridization with the probe.

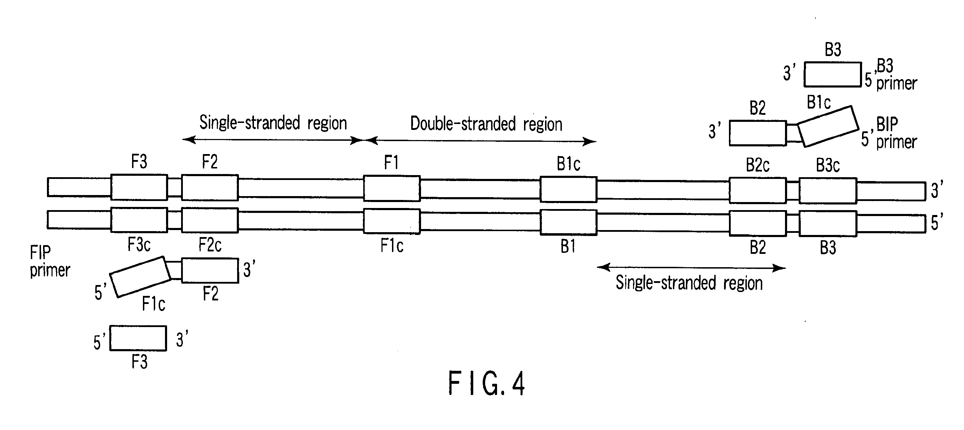

The LAMP method (see, for example, Japanese Patent No. 3313358) is preferably used in amplification of the target nucleic acid. The LAMP method is an easy method yielding an amplification product having a stem loop structure. The principle of the LAMP method is described briefly by reference to the schematic diagram in FIG. 4. In the LAMP method, 4 primers recognizing 6 regions of the target nucleic acid, and a strand displacement-type DNA synthetase, are used. The target nucleic acid is amplified under isothermal conditions (60 to 65°C). The 6 regions are designated F3 region, F2 region and F1 region in the order from the 5'-terminal of the target nucleic acid and B3c region, B2c region and B1c region in the order from the 3'-terminal of the target nucleic acid. The 4 primers have respectively sequences shown in FIG. 4. F1c, F2c, F3c, B1, B2 and B3 regions are regions in chains complementary to the F1, F2, F3, B1c, B2c and B3c regions respectively. In an amplification product by the LAMP method, a loop structure is formed, and regions between F2 and F1 and between B2 and B1 are single-stranded regions. Accordingly, the probe can easily detect the target nucleic acid by utilizing the sequence of this region (see, for example, Jpn. Pat. Appln. KOKAI Publication No. 2005-143492). The reaction time is 30 to 60 minutes, so the target nucleic acid can be rapidly amplified.

### <Reaction conditions>

The binding reaction, which is hybridization between the target nucleic acid and the probe, is carried out under suitable conditions. Suitable conditions vary depending on the type of base contained in the target nucleic acid, the structure of the target nucleic acid and the type of the probe. Suitable conditions are attained for example by a buffer solution having an ionic strength in the range of 0.01 to 5 and a pH value in the range of 5 to 10.

Arbitrary additives may be added to the reaction solution. Examples of the additives include, for example, a hybridization accelerator such as dextran sulfate, salmon sperm DNA, calf thymus DNA, EDTA and a surfactant. The reaction temperature is preferably in the range of 10 to 90°C. The reaction efficiency may be increased by stirring or shaking. A buffer solution having an ionic strength in the range of 0.01 to 5 and a pH value in the range of 5 to 10 may be used in washing after reaction.

### <Probe>

The chain length of the probe, though being not limited, is preferably in the range of 5 to 50 bases, more preferably 10 to 40 bases, still more preferably 15 to 35 bases.

The probe may be unmodified or may be modified with reactive functional groups for immobilization on a support, such as an amino group, carboxyl group, hydroxyl group, thiol group and sulfone group or with substances such as avidin and biotin.

The support for the probe is immobilized may be made of a nitrocellulose membrane, a nylon membrane, a microtiter plate, glass, an electrode, a magnet, beads, plastics, latex, synthetic resin, natural resin or optical fibers.

The support having probes immobilized thereon may be a DNA chip. The DNA chip is a chip having probes immobilized in high density on a substrate such as glass and silicon. The DNA chip can examine sequence information on many genes at one time.

### <Detection method>

Generally, a double-stranded nucleic acid generated by hybridization is detected by a fluorescence detection method. In the method, a gene is labeled with fluorescence and then detected with a highly sensitive fluorescence analyzer. Besides, there is a method of detecting a double-stranded nucleic acid by an electrochemical principle. In this method, a probe is immobilized on an electrode. The double-stranded nucleic acid is electrochemically detected with an electrochemically active intercalator reacting specifically with the double-stranded nucleic acid (see, for example, Jpn. Pat. Appln. KOKAI Publication No. 10-146183). The DNA chip of current detection type neither requires labeling nor needs a large and expensive detector such as in fluorescence detection. Accordingly, this method can be suitably used as an easy and inexpensive method.

Hereinafter, specific examples of the detection method are described in detail.

### 1. Fluorescence detection system

A target nucleic acid is labeled with a fluorescently active substance such as a fluorescent dye. The fluorescent dye includes, but is not limited to, Cy3, Cy5, FITC and rhodamine. Alternatively, a second probe labeled with such substance is used to label the target nucleic acid. The fluorescently labeled target nucleic acid is detected with a suitable detector depending on the type of the label.

### 2. Current detection system

A double-stranded nucleic acid consisting of a target nucleic acid and a probe immobilized on an electrode is detected by binding an intercalator thereto.

The electrode used herein may be produced from, but is not limited to, metal elements such as gold, a gold alloy, silver, platinum, mercury, nickel, palladium, silicon, germanium, gallium, tungsten and alloys thereof, or carbon such as graphite and glassy carbon, or oxides and compounds thereof.

As an intercalator, an electrochemically active substance is used. This substance includes, but is not limited to, Hoechst 33258, acridine orange, quinacrine, daunomycin, a metallointercalator, a bisintercalator such as bisacridine, a trisintercalator and a polyintercalator. These intercalators may be modified with an electrochemically active metal complex such as ferrocene and viologen.

By applying electric potential higher than the electric potential by which the intercalator can electrochemically react, a reaction current value derived from the intercalator can be measured. In this case, the electric potential is swept at constant rate or applied by pulse, or a constant potential may be applied. In measurement, an electric current and electric voltage may be regulated by an apparatus such as a potentiostat, a digital multimeter and a function generator.

In another aspect of the invention, there are provided wild-type and mutant-type probes used in the method of the present invention. There is also provided a probe-immobilized support having these probes immobilized thereon. The probe-immobilized support is provided preferably as a DNA chip. In still another aspect of the invention, there is provided a kit for use in the detection method of the present invention, which comprises the wild-type probe and/or the mutant-type probe as defined above. Preferably, the kit further comprises primers used in LAMP method for amplification of a target nucleic acid. The kit may comprise a strand displacement-type DNA synthetase, a synthetic substrate and a buffer solution.

### EXAMPLES

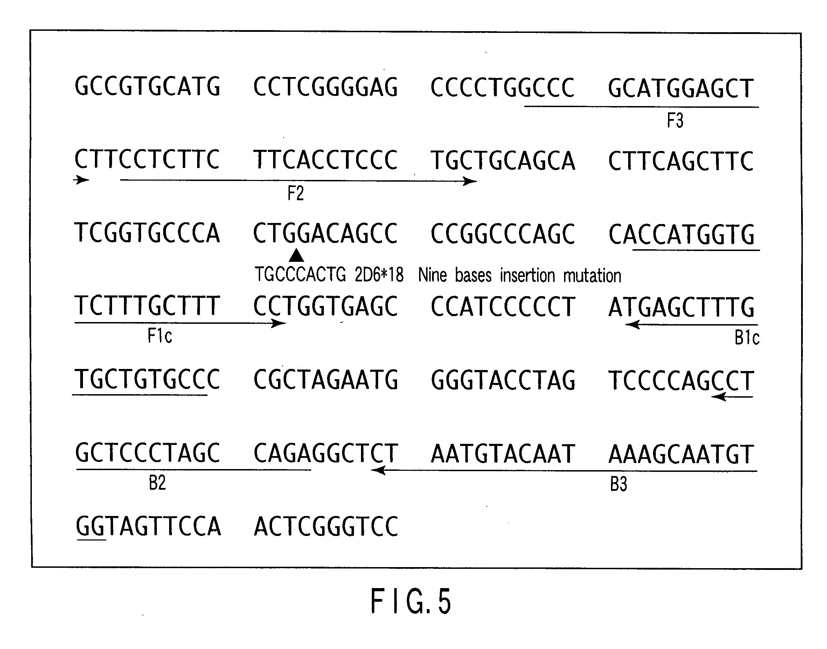

Specific examples of detection by the method of the present invention are described in detail. CYP2D6*18 having a gene insertion mutation with 9 bases in human CYP2D6 gene was used as the target nucleic acid. A partial sequence of the CYP2D6 gene is shown in FIG. 5. A 5.3-kbp CYP2D6 gene fragment was amplified by PCR from heterozygous sample consist of wild-type CYP2D6 and 9-base insertion mutant-type CYP2D6*18. This PCR product was introduced into plasmid vector pCR2.1. The plasmids having the wild-type CYP2D6 or the 9-base insertion mutant-type CYP2D6*18 introduced in were obtained by cloning, respectively. LAMP amplification was carried out using the each plasmid as template respectively. This LAMP product was reacted with a probe immobilized on an electrode. Thereafter, the product bound to the probe was electrochemically detected.

### (1) Amplification of the target nucleic acid

### [Primers]

Primers used in the LAMP method were synthesized. Their sequences are shown below. FIG. 5 shows the positions on the sequence of the target nucleic acid to which the primer's sequence conform to. For F1c region, an opposite strand of the sequence shown in FIG. 5 is used. For B2 and B3 regions, opposite strands of the sequence shown in FIG. 5 are used.
2D6*18 F3 primer: GCCCGCATGGAGCTC 
2D6*18 FIP primer: GGAAAGCAAAGACACCATGGT(F1c)-
CCTCTTCTTCACCTCCCTGC (F2)
 2D6*18 B3 primer: CCACATTGCTTTATTGTACATTAG

2D6*18 BIP primer: TGAGCTTTGTGCTGTGCC(B1c)-
TCTGGCTAGGGAGCAGG (B2)

The FIP primer contains F1c part and F2 part. The BIP primer contains B1c part and B2 part. An arbitrary sequence may or may not be inserted between the parts.

### [Reaction solution]

The reaction solution for LAMP method had the following composition:

| | |
|---|---|
| Sterilized extra-pure water | 5.5 µL |
| Bst DNA polymerase | 1 µL |
| Buffer | 12.5 µL |
| Tris·HCl, pH 8.0 | 40 mM |
| KCI | 20 mM |
| MgSO₄ | 16 mM |
| (NH₄)₂SO₄ | 20 mM |
| Tween 20 | 0.2% |
| Betaine | 1.6 M |
| dNTP | 2.8 mM |
| F3 primer (10 µM) | 0.5 µL |
| B3 primer (10 µM) | 0.5 µL |
| FIP primer (20 µM) | 2 µL |
| BIP primer (20 µM) | 2 µL |
| Template plasmid (3 pg/µl) | 1 µL |
| Total amount | 25 µL |

### [Amplification reaction]

The nucleic acid was amplified by LAMP method at 63°C for 1 hour. As a negative control, sterile water was used in place of the template. The amplified LAMP product was confirmed by agarose gel electrophoresis. As a result, a ladder-shaped pattern typical of the LAMP product appeared. In the negative control, amplification was not observed. It was demonstrated from these results that CYP2D6 could be specifically amplified by using the above primer set.

### (2) Construction of probe-immobilized electrode

### [Probes]

In this example, 5 probes for wild-type detection and 5 probes for insertion-mutation detection were used respectively. For detection of the positive-strand target nucleic acid, the probe is a negative strand. As the negative control, a negative probe having a sequence irrelevant to the CYP2D6 gene sequence was used. The 11 probes mentioned above were modified at the 3'-terminal with thiol for immobilization on a gold electrode.

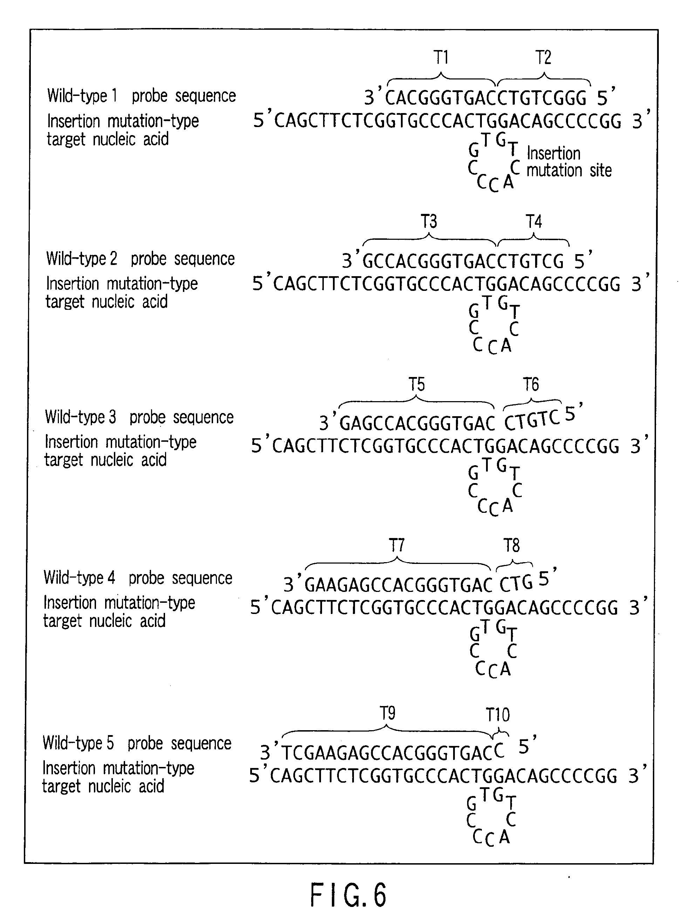

FIG. 6 shows a schematic diagram of the reaction between wild-type probes 1 to 5 and mutant-type target nucleic acid.
- The wild-type probe 1 consists of 18 bases. On the target nucleic acid, the site of a possible inserted mutation is located at the eighth base from the 5'-terminal. The Tm value (T1) of the oligo DNA chain from 3'-terminal to the mutation site was 34°C, and the Tm value (T2) of the oligo DNA chain from 5'-terminal to the mutation site was 28°C, as determined by the Wallace method.
- The wild-type probe 2 consists of 18 bases. On the target nucleic acid, the site of a possible inserted mutation is located at the sixth base from the 5'-terminal. The Tm value (T3) of the oligo DNA chain from 3'-terminal to the mutation site was 42°C and the Tm value (T4) of the oligo DNA chain from 5'-terminal to the mutation site was 20°C.
- The wild-type probe 3 consists of 19 bases. On the target nucleic acid, the site of a possible inserted mutation is located at the fifth base from the 5'-terminal. The Tm value (T5) of the oligo DNA chain from 3'-terminal to the mutation site was 48°C, and the Tm value (T6) of the oligo DNA chain from 5'-terminal to the mutation site was 16°C.
- The wild-type probe 4 consists of 20 bases. On the target nucleic acid, the site of a possible inserted mutation is located at the third base from the 5'-terminal. The Tm value (T7) of the oligo DNA chain from 3'-terminal to the mutation site was 56°C, and the Tm value (T8) of the oligo DNA chain from 5'-terminal to the mutation site was 10°C.
- The wild-type probe 5 consists of 20 bases. On the target nucleic acid, the site of a possible inserted mutation is located at the first base from the 5'-terminal. The Tm value (T9) of the oligo DNA chain from 3'-terminal to the mutation was 62°C, and the Tm value (T10) of the oligo DNA chain from 5'-terminal to the mutation site was 4°C.

The ratio of Tm value of the sequence from 5'-terminal of the probe to the mutation site to Tm value of the sequence from the 3'-terminal of the probe to the mutation site is 1:1.2 for the wild-type probe 1; 1:2.1 for the wild-type probe 2; 1:3.0 for the wild-type probe 3; 1:5.6 for the wild-type probe 4; and 1:15.5 for the wild-type probe 5.

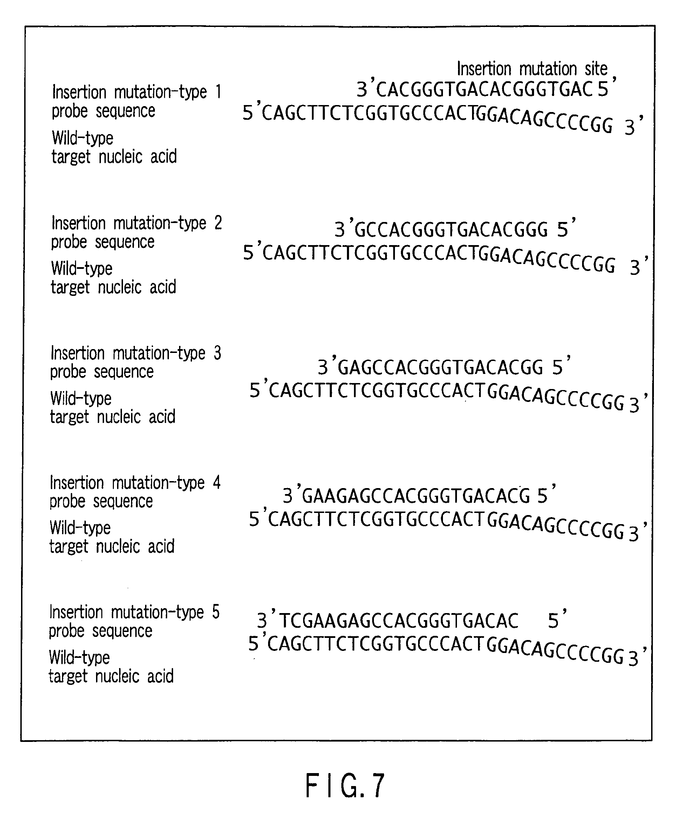

FIG. 7 shows a schematic diagram of the reaction between mutant-type probes 1 to 5 and wild-type target nucleic acid. The mutation sites of all the mutant-type probes 1 to 5 are located in the 5'-terminal side.
- The mutant-type probe 1 consists of 19 bases. The insertion mutation site is in a region from the first to ninth bases from the 5'-terminal.
- The mutant-type probe 2 consists of 17 bases. The insertion mutation site is in a region from the first to fifth bases from the 5'-terminal.
- The mutant-type probe 3 consists of 18 bases. The insertion mutation site is in a region from the first to fourth bases from the 5'-terminal.
- The mutant-type probe 4 consists of 20 bases. The insertion mutation site is in a region from the first to third bases from the 5'-terminal.
- The mutant-type probe 5 consists of 21 bases. The insertion mutation site is in a region from the first to second bases from the 5'-terminal.

Sequences of the respective probes are shown below:
Negative probe: 5'-GTGCTGCAGGTGCG-3'
Wild-type probe1: 5'-GGGCTGTCCAGTGGGCAC-3'
Wild-type probe2: 5'-GCTGTCCAGTGGGCACCG-3'
Wild-type probe3: 5'-CTGTCCAGTGGGCACCGAG-3'
Wild-type 4: 5'-GTCCAGTGGGCACCGAGAAG-3'
Wild-type probe5: 5'-CCAGTGGGCACCGAGAAGCT-3'
Mutant-type probe 1: 5'-CAGTGGGCACAGTGGGCAC-3'
Mutant-type probe 2: 5'-GGGCACAGTGGGCACCG-3'
Mutant-type probe 3: 5'-GGCACAGTGGGCACCGAG-3'
Mutant-type probe 4: 5'-GCACAGTGGGCACCGAGAAG-3'
Mutant-type probe 5: 5'-CACAGTGGGCACCGAGAAGCT-3'

### [Probe-immobilized electrodes]

The above probe was immobilized on a gold electrode via strong covalent bonding between thiol and gold. A solution containing the probe modified at the terminal with thiol was spotted on the gold electrode and left at 25°C for 1 hour. Then, the electrode was dipped in 1 mM mercaptohexanol and washed with 0.2×SSC solution. Each probe was spotted on 2 electrodes. The correspondence of the electrodes to the respective probes is shown below. After washing, the electrode was washed with ultra-pure water and air-dried to give a probe-immobilized electrode.

### [Electrode arrangement]

Electrodes 1, 2: negative probe
Electrodes 3, 4: wild-type probe 1
Electrodes 5, 6: mutant-type probe 1
Electrodes 7, 8: wild-type probe 2
Electrodes 9, 10: mutant-type probe 2
Electrodes 11, 12: wild-type probe 3
Electrodes 13, 14: mutant-type probe 3
Electrodes 15, 16: wild-type probe 4
Electrodes 17, 18: mutant-type probe 4
Electrodes 19, 20: wild-type probe 5
Electrodes 21, 22: mutant-type probe 5

### (3) Hybridization

The probe-immobilized electrode prepared as described above was dipped in the solution containing LAMP product and 2×SSC salt and then left at 55°C for 20 minutes for hybridization. Then, the probe-immobilized electrode was dipped in 0.2×SSC solution at 48°C for 20 minutes and washed. Then, the probe-immobilized electrode was dipped for 10 minutes in a phosphate buffer containing 50 µM Hoechst 33258 as an intercalator. Then, the oxidation current response of Hoechst 33258 was measured.

### (4) Results

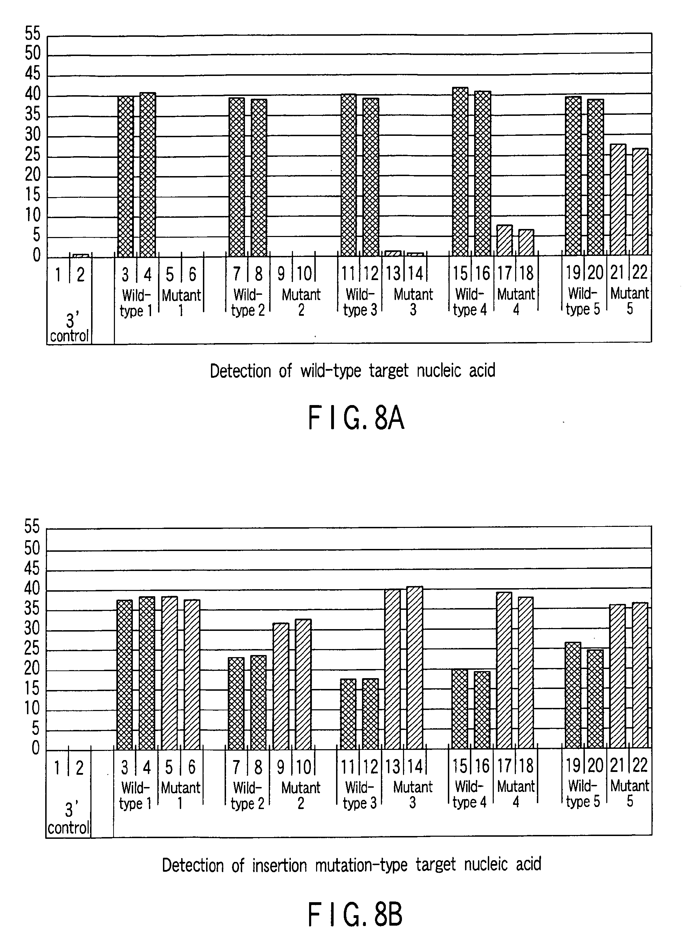

FIG. 8A shows the results of detection of the wild-type target nucleic acid by the wild-type probes 1 to 5 and mutant-type probes 1 to 5. As a matter of course, the target nucleic acid was detected by the wild-type probes 1 to 5. On the other hand, a signal was hardly detected by the mutant-type probes 1 to 3, but a slight signal was detected by the mutant-type probe 4, and a high signal was detected by the mutant-type probe 5. Thus, when the mutation consists of 4 bases or more, unspecific binding is not observed, but when the mutation consists of 3 bases or less, unspecific binding is observed. However, it was shown that unspecific binding can be suppressed by using the mutant-type probe wherein when the number of inserted bases is 3 or less, the ratio of Tm value of the sequence from one end of the probe to the mutation site on the target nucleic acid to Tm value of the sequence from the other end of the probe to the mutation site is 1:2 or more (mutant-type probe 4 and mutant-type probe 5).

FIG. 8B shows the results of detecting the insertion mutant-type target nucleic acid. As a matter of course, the target nucleic acid was detected by the mutant-type probes 1 to 5. On the other hand, a very high signal was detected by the mutant-type probe 1 (Tm ratio of 1:1.2) which the site of insertion mutation was located in almost the center of the probe. No difference in signal between the wild-type probe 1 and mutant-type probe 1 was recognized, thus indicating that there are many unspecific bindings.

In the wild-type probe 2 (Tm ratio of 1:2.1), which the site of the insertion mutation was put slightly towards the terminal, there was difference from the mutant-type probe 2, and the mutation could be detected. In the wild-type probe 3 (Tm ratio of 1:3.0) and the wild-type probe 4 (Tm ratio of 1:5.6) which the mutation site was further put towards the terminal, there was a significant difference from the mutant-type probes 3 and 4. In the wild-type probe 5 which the mutation site was put to the terminal, there was also a difference from the mutant-type probe 5 (Tm ratio of 1:15.5).

Accordingly, it was revealed that when the mutation site is located outside of the center of the probe, unspecific signal can be eliminated. Specifically, it was revealed that when the wild-type probe has the ratio of Tm value of the sequence from one end of the probe to the mutation site on the target nucleic acid to Tm value of the sequence from the other end of the probe to the mutation site is 1:2 or more, the unspecific binding between the wild-type probe and mutant-type target nucleic acid can be eliminated.

In the Examples above, an experiment for detection of an insertion mutation was carried out, but it would be easily recognized that with respect to detection of a deletion mutation, unspecific binding is suppressed by the probe with the opposite action, thereby enabling accurate detection of the mutation.

For example, the binding between a deletion mutant-type target nucleic acid and a wild-type probe can be understood in the same manner as in the binding between a wild-type target nucleic acid and a mutant-type probe for insertion mutation. In addition, the binding between a wild-type target nucleic acid and a mutant-type probe for deletion mutation can also be understood in the same manner as in the binding between an insertion mutant-type target nucleic acid and a wild-type probe. It follows that in detection of a deletion mutation, unspecific binding can be reduced and the deletion mutation can be detected accurately by using a mutant-type probe which the ratio of Tm value of the sequence from one end of the probe to the mutation site to Tm value of the sequence from the other end of the probe to the mutation site is 1:2 or more, and, a wild-type probe which, when the number of deleted bases is 3 or less, the ratio of Tm value of the sequence from one end of the probe to the mutation site to Tm value of the sequence from the other end of the probe to the mutation site is 1:2 or more.

## Claims

1. A method of detecting a gene insertion mutation, comprising the steps of:
reacting a target nucleic acid with a wild-type probe and a mutant-type probe,
detecting the amount of the target nucleic acid bound to said each probes, and
comparing the amount of the target nucleic acid bound to the wild-type probe with the amount of the target nucleic acid bound to the mutant-type probe,
**characterized in that** the wild-type probe has a sequence complementary to a sequence of the wild-type target nucleic acid, and the ratio of Tm value of the sequence from one end of the probe to the mutation site to Tm value of the sequence from the other end of the probe to the mutation site is 1:2 or more.

2. The method according to claim 1, **characterized in that** the mutant-type probe has a sequence complementary to a sequence of the mutant-type target nucleic acid, and when the number of inserted bases is 3 or less, the ratio of Tm value of the sequence from one end of the probe to the inserted bases to Tm value of the sequence from the other end of the probe to the inserted bases is 1:2 or more.

3. The method according to claim 1, **characterized in that** the target nucleic acid has a stem loop structure, and if the target nucleic acid has a mutation, the mutation is located in the part of the loop structure.

4. The method according to claim 3, **characterized in that** the target nucleic acid is a product amplified by LAMP method.

5. The method according to claim 1, **characterized in that** the difference between Tm value of the wild-type probe and the mutant-type probe is 10°C or smaller.

6. The method according to claim 1, **characterized in that** the probe is immobilized on a support.

7. The method according to claim 1, **characterized in that** the target nucleic acid bound to the probe is detected with an electrochemically active nucleic acid-recognizing body.

8. A method of detecting a gene deletion mutation, comprising the steps of:
reacting a target nucleic acid with a wild-type probe and a mutant-type probe,
detecting the amount of the target nucleic acid bound to each of the probes, and
comparing the amount of the target nucleic acid bound to the wild-type probe with the amount of the target nucleic acid bound to the mutant-type probe,
**characterized in that** the mutant-type probe has a sequence complementary to a sequence of the mutant-type target nucleic acid, and the ratio of Tm value of the sequence from one end of the probe to the deleted bases to Tm value of the sequence from the other end of the probe to the deleted bases is 1:2 or more.

9. The method according to claim 8, **characterized in that** the wild-type probe has a sequence complementary to a sequence of the wild-type target nucleic acid, and when the number of deleted bases is 3 or less, the ratio of Tm value of the sequence from one end of the probe to the mutant site to Tm value of the sequence from the other end of the probe to the mutant site is 1:2 or more.

10. The method according to claim 8, **characterized in that** the target nucleic acid has a stem loop structure, and if the target nucleic acid has a mutation, the mutation is located in the part of the loop structure.

11. The method according to claim 10, **characterized in that** the target nucleic acid is a product amplified by LAMP method.

12. The detection method according to claim 8, **characterized in that** the difference between Tm value of the wild-type probe and the mutant-type probe is 10°C or smaller.

13. The method according to claim 8, **characterized in that** the probe is immobilized on a support.

14. The method according to claim 8, **characterized in that** the target nucleic acid bound to the probe is detected with an electrochemically active nucleic acid-recognizing body.


SEQUENCE LISTING

<110> Kabushiki Kaisha Toshiba

<120> Method of Detection of Insert and Deletion Mutation

<130> 07S0978

<140>
<141>

<150> JP 2007-031318
<151> 2007-02-09

<160> 31

<170> PatentIn version 3.3

<210> 1
<211> 15
<212> DNA
<213> Artificial

<220>
<223> F3 Primer

<400> 1
gcccgcatgg agctc 15

<210> 2
<211> 21
<212> DNA
<213> Artificial

<220>
<223> FIP Primer (F1c)

<400> 2
ggaaagcaaa gacaccatgg t 21

<210> 3
<211> 20
<212> DNA
<213> Artificial

<220>
<223> FIP Primer (F2)

<400> 3
cctcttcttc acctccctgc 20

<210> 4
<211> 24
<212> DNA
<213> Artificial

<220>
<223> B3 Primer

<400> 4
ccacattgct ttattgtaca ttag 24

<210> 5
<211> 18
<212> DNA
<213> Artificial

<220>
<223> BIP Primer (B1c)

<400> 5
tgagctttgt gctgtgcc 18

<210> 6
<211> 17
<212> DNA
<213> Artificial

<220>
<223> BIP Primer (B2)

<400> 6
tctggctagg gagcagg 17

<210> 7
<211> 14
<212> DNA
<213> Artificial

<220>
<223> Negative Probe

<400>  7
gtgctgcagg tgcg                                                    14

<210>  8
<211>  18
<212>  DNA
<213>  Artificial

<220>
<223>  Wild-type probe-1

<400>  8
gggctgtcca gtgggcac                                                18

<210>  9
<211>  19
<212>  DNA
<213>  Artificial

<220>
<223>  Mutation-type probe-1

<400>  9
cagtgggcac agtgggcac                                               19

<210>  10
<211>  18
<212>  DNA
<213>  Artificial

<220>
<223>  Wild-type probe-2

<400>  10
gctgtccagt gggcaccg                                                18

<210>  11
<211>  17
<212>  DNA
<213>  Artificial

<220>
<223>  Mutation-type probe-2

<400> 11
gggcacagtg ggcaccg 17

<210> 12
<211> 19
<212> DNA
<213> Artificial

<220>
<223> Wild-type probe-3

<400> 12
ctgtccagtg ggcaccgag 19

<210> 13
<211> 18
<212> DNA
<213> Artificial

<220>
<223> Mutation-type probe-3

<400> 13
ggcacagtgg gcaccgag 18

<210> 14
<211> 20
<212> DNA
<213> Artificial

<220>
<223> Wild-type probe-4

<400> 14
gtccagtggg caccgagaag 20

<210> 15
<211> 20
<212> DNA
<213> Artificial

<220>
<223> Mutation-type probe-4

<400> 15
gcacagtggg caccgagaag 20

<210> 16
<211> 20
<212> DNA
<213> Artificial

<220>
<223> Wild-type probe-5

<400> 16
ccagtgggca ccgagaagct 20

<210> 17
<211> 21
<212> DNA
<213> Artificial

<220>
<223> Mutation-type probe-5

<400> 17
cacagtgggc accgagaagc t 21

<210> 18
<211> 260
<212> DNA
<213> Homo sapiens

<220>
<223> Wild-type CYP2D6

<400> 18
gccgtgcatg cctcggggag cccctggccc gcatggagct cttcctcttc ttcacctccc 60

tgctgcagca cttcagcttc tcggtgccca ctggacagcc ccggcccagc caccatggtg 120

tctttgcttt cctggtgagc ccatccccct atgagctttg tgctgtgccc cgctagaatg 180

gggtacctag tccccagcct gctccctagc cagaggctct aatgtacaat aaagcaatgt 240

ggtagttcca actcgggtcc 260

<210> 19
<211> 269
<212> DNA
<213> Homo sapiens

<220>
<223> Mutation-type CYP2D6

<400> 19
gccgtgcatg cctcggggag cccctggccc gcatggagct cttcctcttc ttcacctccc 60

tgctgcagca cttcagcttc tcggtgccca ctgtgcccac tggacagccc cggcccagcc 120

accatggtgt ctttgctttc ctggtgagcc catcccccta tgagctttgt gctgtgcccc 180

gctagaatgg ggtacctagt ccccagcctg ctccctagcc agaggctcta atgtacaata 240

aagcaatgtg gtagttccaa ctcgggtcc 269

<210> 20
<211> 40
<212> DNA
<213> Homo sapiens

<220>
<223> Mutation-type target sequence

<400> 20
cagcttctcg gtgcccactg tgcccactgg acagccccgg 40

<210> 21
<211> 18
<212> DNA
<213> Homo sapiens

<220>
<223> Wild type-1 probe

<400> 21
gggctgtcca gtgggcac 18

<210> 22
<211> 18
<212> DNA
<213> Artificial

<220>
<223>  Wild type-2 probe

<400>  22
gctgtccagt gggcaccg                                                    18


<210>  23
<211>  19
<212>  DNA
<213>  Artificial

<220>
<223>  Wild type-3 probe

<400>  23
ctgtccagtg ggcaccgag                                                   19


<210>  24
<211>  20
<212>  DNA
<213>  Artificial

<220>
<223>  Wild type-4 probe

<400>  24
gtccagtggg caccgagaag                                                  20


<210>  25
<211>  20
<212>  DNA
<213>  Artificial

<220>
<223>  Wild type-5 probe

<400>  25
ccagtgggca ccgagaagct                                                  20


<210>  26
<211>  31
<212>  DNA
<213>  Homo sapiens

<220>

<223> Wild-type target sequence

<400> 26
cagcttctcg gtgcccactg gacagccccg g 31

<210> 27
<211> 19
<212> DNA
<213> Artificial

<220>
<223> Mutation type-1 probe

<400> 27
cagtgggcac agtgggcac 19

<210> 28
<211> 17
<212> DNA
<213> Artificial

<220>
<223> Mutation type-2 probe

<400> 28
gggcacagtg ggcaccg 17

<210> 29
<211> 18
<212> DNA
<213> Artificial

<220>
<223> Mutation type-3 probe

<400> 29
ggcacagtgg gcaccgag 18

<210> 30
<211> 20
<212> DNA
<213> Artificial

<220>
<223> Mutation type-4 probe

<400>  30
gcacagtggg caccgagaag                                                    20

<210>  31
<211>  21
<212>  DNA
<213>  Artificial

<220>
<223>  Mutation type-5 probe

<400>  31
cacagtgggc accgagaagc t                                                  21